# EUROPEAN PATENT APPLICATION

(11) **EP 4 015 497 A1**
(43) Date of publication of application: **22.06.2022**
(21) Application number: 20215421.7
(22) Date of filing: 18.12.2020
(51) Int. Cl.: C07C 41/03, C07C 43/11, C07C 43/13, C07C 43/23, C11D 1/06, C11D 1/72

(54) **ALKOXYLATES PREPARED BY CALCIUM CATALYST**

(71) Applicant: Clariant International Ltd, 4132 Muttenz (CH)
(72) Inventor: GUO, Xiaoqiang, 65926 Frankfurt am Main (DE); LEINWEBER, Dirk, 65926 Frankfurt am Main (DE); DIEDERICHS, Jan, 84508 Burgkirchen a. d. Alz (DE); HÖVELMANN, Felix, 84508 Burgkirchen a. d. Alz (DE); BATCHELOR, Stephen Norman, Wirral, Merseyside, CH63 3JW (GB); BENNETT, Julie, Wirral, Merseyside, CH63 3JW (GB)
(74) Representative: Paczkowski, Marcus

(57) **Abstract**

The present invention relates to a fatty alcohol alkoxylate surfactant, obtainable by reacting at least one fatty alcohol with ethylene oxide or a mixture of ethylene oxide and propylene oxide, wherein the reaction is conducted in the presence of a calcium catalyst. Furthermore, the present invention refers to a fatty alcohol alkoxylate surfactant comprising at least one fatty alcohol alkoxylate and at least one calcium salt. The present invention further refers to a method for preparing a fatty alcohol alkoxylate surfactant according to the invention and the use of a calcium catalyst for preparing such a fatty alcohol alkoxylate surfactant.

## Description

The present invention relates to a fatty alcohol alkoxylate surfactant, obtainable by reacting at least one fatty alcohol with ethylene oxide or a mixture of ethylene oxide and propylene oxide, wherein reacting is conducted in the presence of a calcium catalyst. Furthermore, the present invention refers to a fatty alcohol alkoxylate surfactant comprising at least one fatty alcohol alkoxylate and at least one calcium salt. The present invention further refers to a method for preparing a fatty alcohol alkoxylate surfactant according to the invention and the use of a calcium catalyst for preparing such fatty alcohol alkoxylate surfactant.

Today, surfactant compositions are of interest for a large number of uses. For instance, consumer goods such as, shampoos, shower gels, toothpastes, laundry formulations, etc., as well as industrial use such as for production processes including emulsification of immiscible phases, are of great interest. For many applications, non-ionic surfactants are desired. Often, non-ionic surfactants are less sensitive to water hardness than, for instance, many anionic surfactants. Non-ionic surfactants such as alkoxylates often exhibit good foaming properties and/or good emulsification properties as desired for many uses. A frequently used class of non-ionic surfactants is the group of alkoxylate surfactants such as, e.g., alcohol ethoxylate surfactants.

Typically, alkoxylate surfactants are obtained by reacting an alcohol with the desired molar ratio of alkylene oxide (often ethylene oxide). This typically requires heating the reagents in the presence of a catalyst.

A common catalyst in this context is a strong inorganic base such as sodium hydroxide. This has several technical drawbacks. In the production process, the operator has to deal with considerable amounts of a harmful strong inorganic base such as sodium hydroxide. Furthermore, the obtained product is basic and has to be neutralized. In the basic environment, undesired saponification may occur when the ester compounds are present.

A severe drawback of the synthetic route of the prior art is that an undesirably broad distribution of the number of alkoxy residues per alcohol group is obtained. In other words, the obtained alkoxylate surfactant has a poor homology. It is a mixture of a large variety of alkoxylate with different properties. This may hamper usability of the surfactant and may even result in undesired phase separation upon storage. The properties as well as the reliability of the alkoxylate surfactants in the art is not as desired.

Antimony pentachloride was described as an alternative catalyst for catalyzing the reaction of an alcohol ethylene oxide as described in GB-A 796,508. Antimony salts are, however, considered as being harmful or even toxic. Thus, the method and alkoxylate surfactants described in GB-A 796,508 have significant drawbacks.

EP-A 0026546 teaches the reaction of hydroxyl group-containing reactants with epoxides in the presence of a basic salt of an alkaline earth metal. The disclosure of EP-A 0026546 is focused on basic earth alkali metal alkoxides, in particular earth alkali metal alkoxides ethoxides and methoxides prepared by reacting the earth alkali metal with ethanol. For instance, basic calcium ethoxides obtained from the reaction of calcium metal with ethanol are used as catalyst. Also EP-A 0092256 teaches earth alkali metal alkoxides such as, e.g., calcium alkoxides, usable as catalyst for preparing alkoxides and adds a catalyst promoter selected from a long list of alternatives. Using alkali metal alkoxides such as, e.g., calcium alkoxides, bears the advantage that catalysis is performed rather well. A severe drawback is, however, that alkali metal alkoxides are chemically not well storable and are, thus, typically to be prepared on demand on site. Preparing the catalyst is laborious as it requires constantly removing the ethanol by-product by means of evaporation in a vacuum. This procedure is rather laborious and far from being optimal.

In view of the prior art, there is still an unmet need for alkoxylate surfactants having improved properties such as good solubility, absence of undesired basic or toxic components, good synthetic accessibility and narrow distribution of the number of alkoxy residues per alcohol group. Further, there is also the desire for effective processes for obtaining such alkoxylate surfactants and the avoidance of non-storable educts and catalysts.

Surprisingly, it was found that a fatty alcohol alkoxylate surfactant having particularly good properties such as narrow distribution of the number of alkoxy residues per alcohol group and good solubility can be obtained by using a calcium catalyst. Using such calcium catalyst allows the avoidance of toxic and basic reagents and the calcium catalyst can optionally partly or completely remain in the fatty alcohol alkoxylate surfactant without disturbing its usability for many applications.

A first aspect relates to a fatty alcohol alkoxylate surfactant, obtainable (or obtained) by reacting at least one fatty alcohol with ethylene oxide or a mixture of ethylene oxide and propylene oxide, wherein the reaction is conducted in the presence of a calcium catalyst.

As used herein, the term "surfactant" may be understood in the broadest sense as generally understood in the art. Typically, it is a surface-active agent that is able to lower the surface tension of aqueous solutions. A surfactant may act as a detergent, wetting agent, emulsifier, foaming agent, and/or dispersant. In a preferred embodiment, a surfactant as used herein is a non-ionic surfactant.

As used herein, propylene oxide is typically 1,2-propylene oxide.

Reacting the at least one fatty alcohol with ethylene oxide may lead to a fatty alcohol residue that is conjugated with one or more ethoxy groups. Reacting the at least one fatty alcohol with propylene oxide may lead to a fatty alcohol residue that is conjugated with one or more propoxy groups. Reacting the at least one fatty alcohol with ethylene oxide and propylene oxide may lead to a fatty alcohol residue that is conjugated with one or more ethoxy groups and one or more propoxy groups.

When a mixture of ethylene oxide and propylene oxide are used, any molar ratio may be used. In a preferred embodiment, the mixture of ethylene oxide and propylene oxide has molar ratio of (ethylene oxide) : (propylene oxide) of > 1:1.

In a preferred embodiment, the mixture of ethylene oxide and propylene oxide has a molar ratio of (ethylene oxide) : (propylene oxide) of > 1.1:1, > 1.2:1, > 1.5:1, > 2:1, > 3:1, or > 5:1.

As used throughout the present invention, the term "ethoxy" may be understood as generally understood in the art. In the context of a residue embedded in a chemical structure, it may have the structure

[#-CH₂-CH₂-O-#]ₙ,

wherein each # independently from one another is a binding side to another residue of the compound or, at its terminal end, to hydrogen, and n is the number of ethoxy residues.

As used throughout the present invention, the term "propoxy" may be understood as generally understood in the art. In the context of a residue embedded in a chemical structure, preferably has any of the structures:

[#-CH(CH₃)-CH₂-O-#]ₙ,

or

[#-CH₂-CH(CH₃)-O-#]ₙ.

wherein each # independently from one another is a binding side to another residue of the compound or, at its terminal end, to hydrogen, and n is the number of propoxy residues. In a preferred embodiment, in the context of a residue embedded in a chemical structure, it may have any of the structures [#-CH(CH₃)-CH₂-O-#]ₙ, or [#-CH₂-CH(CH₃)-O-#]ₙ, in particular is [#-CH₂-CH(CH₃)-O-#]ₙ.

A fatty alcohol alkoxylate surfactant as used herein may be understood in the broadest sense as a conjugate of a fatty alcohol group and one or more alkoxy residues. Typically, the bond between the fatty alcohol group and the one or more alkoxy residues is an ether bond (-O-). Also the bond between two or more alkoxy residues is typically each an ether bond.

The term "fatty alcohol" may be understood in the broadest sense as generally understood in the art. Preferably, it is a linear or branched, saturated or unsaturated (e.g. comprising one, two, three or more than three double bonds) alcohol having 4 to 34 carbon atoms, preferably 4 to 26 carbon atoms, more preferably 6 to 22 carbon atoms. For instance, a fatty alcohol may have 6, 7, 8, 9, 10, 11, 12, 13, 14, 15, 16, 17, 18, 19, 20, 21, or 22 carbon atoms. In a preferred embodiment, the fatty alcohol is an alkyl group comprising a single hydroxyl group. In a preferred embodiment, the fatty alcohol is an alkyl group comprising a single hydroxyl group at carbon atom C₁, i.e., a terminal carbon atom. In a preferred embodiment, the fatty alcohol has a linear structure.

For instance, a fatty alcohol may be selected from the group consisting of butyl alcohol (e.g., tert-butyl alcohol), tert-amyl alcohol, 3-methyl-3-pentanol, hexanol (e.g., 1-hexanol), heptanol (e.g., 1-heptanol, enanthic alcohol), octanol (e.g., 1-octanol, capryl alcohol), nonanol (e.g. 1-nonanol, pelargonic alcohol), decanol (e.g., 1-decanolm, decyl alcohol, capric alcohol), undecyl alcohol (e.g., 1-undecanol, hendecanol), dodecanol (e.g., 1-dodecanol, lauryl alcohol), tridecyl alcohol (e.g., 1-tridecanol, isotridecanol), tetradecanol (e.g., 1-tetradecanol, myristyl alcohol), pentadecanol, (e.g., 1-pentadecanol, pentadecanol), hexadecanoyl (e.g., 1-hexadecanol, cetyl alcohol), palmitoleyl alcohol (cis-9-hexadecen-1-ol), heptadecanol (e.g., 1-n-heptadecanol, heptadecyl alcohol),
octadecanol (e.g., 1-octadecanol, stearyl alcohol), octadecenol (e.g. 1-octadecenol, oleyl alcohol, octadec-9-en-1-ol), nonadecanol (e.g., 1-nonadecanol, nonadecyl alcohol), eicosanol (e.g., 1-eicosanol, arachidyl alcohol), heneicosanol (e.g., 1-heneicosanol, heneicosyl alcohol), docosanol (e.g., 1-docosanol, behenyl alcohol), cis-13-docosen-1-ol (erucyl alcohol), tetracosanol (e.g., 1-tetracosanol (lignoceryl alcohol), hexacosanol (e.g., 1-hexacosanol, ceryl alcohol), octacosanol, nonacosanol, dotriacontanol, and tetratriacontanol.

In a preferred embodiment, a fatty alcohol may be selected from the group consisting of 1-n-hexadecanol, 9-hexadecen-1-ol, 1-n-heptadecanol, 1-n-octadecanol, 1-n-octadecenol (e.g., octadec-9-en-1-ol), 1-n-nonadecanol, and eicosanol (e.g., 1-n-eicosanol). In a preferred embodiment, a fatty alcohol may be octadec-9-en-1-ol.

In a preferred embodiment, a fatty acid residue incorporated in an alkoxylate has the structure

R^{a}-O-#,

wherein
- #: is the binding side to a carbon atom of the one or more alkoxide residues; and
- R^{a}: is a linear or branched C₄-C₃₄-alkyl or C₄-C₃₄-alkenyl, preferably a linear or branched C₆-C₂₀-alkyl or C₆-C₂₀-alkenyl, more preferably a linear or branched C₈-C₂₀-alkyl or C₈-C₂₀-alkenyl, even more preferably a C₁₂-C₂₀-alkyl or C₁₂-C₂₀-alkenyl, even more preferably a linear or branched C₁₆-C₁₈-alkyl or C₁₆-C₁₈-alkenyl,

In a preferred embodiment, R^{a} is a linear alkyl or alkenyl residue, in particular a linear C₁₆-C₁₈-alkyl or C₁₆-C₁₈-alkenyl.

As used throughout the present invention, the terms "alkyl" and "alkenyl" may be understood in the broadest sense as generally understood in the art.

An alkyl group is mainly or completely composed of carbon and hydrogen atoms and does not contain a double bond. An alkyl group may be linear (also: unbranched) or branched. It will be understood that a branched alkyl comprises at least three carbon atoms. Thus, for instance, in a branched or linear C₁-C₂₀-alkyl, the person skilled in the art will understand without any burden that a linear alkyl may have from 1 to 20 carbon atoms, while a branched alkyl may have from 3 to 20 carbon atoms.

An alkenyl group is mainly or completely composed of carbon and hydrogen atoms and contains one, two, three or even more than three double bonds. Typically and preferably, a single carbon atom is not involved in two double bonds. An alkenyl group may be linear or branched. It will be understood that a branched alkenyl comprises at least three carbon atoms. Thus, for instance, in a branched or linear C₂-C₂₀-alkenyl, the person skilled in the art will understand without any burden that a linear alkenyl may have from 2 to 20 carbon atoms, while a branched alkenyl may have from 3 to 20 carbon atoms.

Alkyl or alkenyl groups may or may not be substituted by one or more heteroatoms, preferably are not substituted by a heteroatom. As used throughout the present invention, the term "carbon atom" may include any isotope of carbon, including ¹²C, ¹³C and ¹⁴C, preferably is ¹²C and/or ¹³C. As used throughout the present invention, the term "hydrogen" may include any isotope of hydrogen including ¹H, ²H (deuterium) and ³H (tritium).

### (A) Fatty alcohol alkoxylate

A fatty alcohol alkoxylate used in the present invention may be any fatty alcohol alkoxylate that is obtainable by reacting at least one fatty alcohol with ethylene oxide or a mixture of ethylene oxide and propylene oxide, wherein the reaction is conducted in the presence of a calcium catalyst.

In a preferred embodiment, the fatty alcohol alkoxylate surfactant comprises at least one fatty alcohol alkoxylate described in the following formula (I) 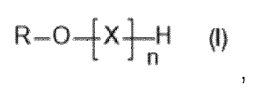 wherein:,
- R: is a linear or branched alkyl or alkenyl group having 6 to 20 carbon atoms, preferably a linear or branched alkyl or alkenyl group having 8 to 20 carbon atoms, more preferably a linear or branched alkyl or alkenyl group having 12 to 20 carbon atoms, even more preferably a linear or branched alkyl or alkenyl group having 16 to 18 carbon atoms, in particular a linear alkyl or alkenyl group having 16 to 18 carbon atoms;
- X: is selected from ethoxy and mixtures of ethoxy and propoxy, wherein a mixture of ethoxy and propoxy has molar ratio of ethoxy : propoxy of > 1:1, in particular is ethoxy;
- n: on a molar average, is from 3 to 100, preferably from 3 to 60, more preferably from 3 to 40, even more preferably from 4 to 20, in particular from 4 to 15.

In a preferred embodiment, R is a linear alkyl or alkenyl group.

In a preferred embodiment, X is ethoxy. In another preferred embodiment, X is a mixture of ethoxy and propoxy having a molar ratio of ethoxy : propoxy of > 1:1, > 1.1:1, > 1.2:1, > 1.5:1, >2:1, >3:1, or > 5:1.

In a preferred embodiment, on a molar average, n is from 4 to 15, from 4 to 10, from 5 to 12, from 7 to 15, or from 10 to 15.

In a preferred embodiment, in more than 70 mol% of the fatty alcohol alkoxylates, n is in the range from 0.5·n to 1.5·n. Thus, the distribution of n (i.e., the ethoxy and optionally propoxy residues) is rather narrow on molar average. In other words, preferably in more than 70 mol% of the fatty alcohol alkoxylates, n is in the range from +/- 0.5·n.

In a preferred embodiment, in more than 75 mol% or more than 80 mol% of the fatty alcohol alkoxylates, n is in the range from 0.5·n to 1.5·n.

In a preferred embodiment, in the at least one fatty alcohol alkoxylate of formula (I):
- R: is a linear alkyl or alkenyl group having 16 to 18 carbon atoms;
- X: is ethoxy; and
- n: on a molar average, is from 4 to 20, in particular from 4 to 15,
in particular wherein in more than 70 mol% of the fatty alcohol alkoxylates, n is in the range from 0.5·n to 1.5·n.

The fatty alcohol alkoxylate surfactant may comprise the fatty alcohol alkoxylate in any content. In a preferred embodiment, the fatty alcohol alkoxylate surfactant of the present invention comprises fatty alcohol alkoxylate, preferably the one or more fatty alcohol alkoxylate of formula (I), in an amount from 0.1 to 99.99% by weight, preferably in an amount from 1 to 99.9% by weight, more preferably in an amount from 10 to 99.9% by weight and even more preferably in an amount from 50 to 99.9%, in each case based on the total weight of the fatty alcohol alkoxylate surfactant.

As noted above, a technical advantage of many preferred embodiment of the present invention is that using a calcium catalyst enables that it may partly or completely remain in the fatty alcohol alkoxylate surfactant without hampering the usability of the fatty alcohol alkoxylate surfactant. Accordingly, the fatty alcohol alkoxylate surfactant may optionally contain the calcium catalyst. The fatty alcohol alkoxylate surfactant may comprise the calcium catalyst in any content. In a preferred embodiment, the fatty alcohol alkoxylate surfactant according to the present invention comprises the one or more calcium catalysts in an amount from 0.01 to 10% by weight, preferably in an amount from 0.01 to 5% by weight, more preferably in an amount from 0.05 to 3% by weight and even more preferably in an amount from 0.1 to 2%, in each case based on the total weight of the fatty alcohol alkoxylate surfactant.

In a preferred embodiment, the fatty alcohol alkoxylate surfactant of the present invention comprises:
(A) the one or more fatty alcohol alkoxylates, preferably the one or more fatty alcohol alkoxylate of formula (I), in an amount from 0.1 to 99.99% by weight, preferably in an amount from 1 to 99.9% by weight, more preferably in an amount from 10 to 99.9% by weight and even more preferably in an amount from 50 to 99.9%, in each case based on the total weight of the fatty alcohol alkoxylate surfactant; and
(B) the one or more calcium catalysts in an amount from 0.01 to 10% by weight, preferably in an amount from 0.01 to 5% by weight, more preferably in an amount from 0.05 to 3% by weight and even more preferably in an amount from 0.1 to 2%, in each case based on the total weight of the fatty alcohol alkoxylate surfactant; and
(C) optionally water; and
(D) optionally further ingredients usable in a surfactant composition.

The one or more calcium catalysts and the fatty alcohol alkoxylate may be used in any weight ratio. In a preferred embodiment, the weight ratio of (fatty alcohol alkoxylate) : (one or more calcium catalysts) is from 98.2 : 1.8 to 99.99 : 0.01, more preferably from 98.5 : 1.5 to 99.9 :0.1 and in particular from 99.0 : 1.0 to 99.8 : 0.2.

### (B) Calcium catalyst

The calcium catalyst may be any catalyst that comprises calcium. In a preferred embodiment, the calcium catalyst comprises or consist of at least one calcium salt, i.e., at least one salt comprising calcium cations (Ca²⁺) and one or more types of anions as counterions. It will be understood that, optionally, such calcium salt may also form part of a mixture.

In a preferred embodiment, the calcium catalyst comprises or consist of at least one calcium salt which comprises calcium cations and one or more types of anions selected from the group consisting of branched or linear C₈-C₁₂-alkanoate (preferably branched C₈-C₁₂-alkanoate, in particular branched nonanoate), hydrogensulfate, sulfate an anionic ion of formula (III), and an anionic ion of formula (IV) 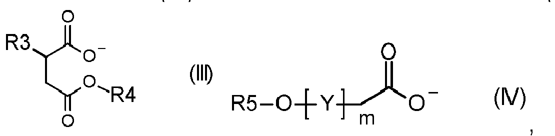 wherein
- R3: is a linear or branched alkyl group having 1 to 20 carbon atoms or a linear or branched alkenyl group having 2 to 20 carbon atoms, preferably a linear or branched alkyl or alkenyl group having 6 to 18 carbon atoms, in particular a linear or branched alkyl or alkenyl group having 8 to 16 carbon atoms;
- R4: is a linear or branched alkyl group having 1 to 20 carbon atoms or a linear or branched alkenyl group having 2 to 20 carbon atoms, preferably a linear or branched alkyl group having 1 to 16 carbon atoms or a linear or branched alkenyl group having 2 to 16 carbon atoms, in particular a linear or branched alkyl group having 1 to 12 carbon atoms or a linear or branched alkenyl group having 2 to 12 carbon atoms;
- R5: is a linear or branched alkyl group having 1 to 20 carbon atoms or a linear or branched alkenyl group having 2 to 20 carbon atoms, preferably a linear or branched alkyl or alkenyl group having 6 to 20 carbon atoms, in particular a linear or branched alkyl or alkenyl group having 10 to 20 carbon atoms;
- Y: is selected from ethoxy and mixtures of ethoxy and propoxy, wherein a mixture of ethoxy and propoxy has molar ratio of ethoxy : propoxy of > 1:1, in particular is ethoxy; and
- m: on a molar average, is from 3 to 100, preferably from 3 to 40, more preferably from 3 to 20, in particular from 3 to 10.

In a preferred embodiment, the residues in formulae (III) and/or (IV) are defined as follows:
- R3: is a linear or branched alkyl or alkenyl group having 8 to 16 carbon atoms;
- R4: is a linear or branched alkenyl group having 2 to 12 carbon atoms;
- R5: is a linear or branched alkyl or alkenyl group having 10 to 20 carbon atoms;
- Y: is selected from ethoxy; and
- m: on a molar average, is from 3 to 10.

In a preferred embodiment, R3 is a linear alkyl group having 8, 9, 10, 11, 12, 13, 14, 15 or 16 carbon atoms. In another preferred embodiment, R3 is a branched alkyl group having 8, 9, 10, 11, 12, 13, 14, 15 or 16carbon atoms.

In a preferred embodiment, R4 is a linear alkyl group having 2, 3, 4, 5, 6, 7, 8, 9, 10, 11 or 12 carbon atoms. In another preferred embodiment, R4 is a branched alkyl group having 3, 4, 5, 6, 7, 8, 9, 10, 11 or 12 carbon atoms.

In a preferred embodiment, R5 is a linear alkyl group having 10, 11, 12, 13, 14, 15, 16, 17, 18, 19 or 20 carbon atoms. In another preferred embodiment, R5 is a branched alkyl group having 10, 11, 12, 13, 14, 15, 16, 17, 18, 19 or 20 carbon atoms.

In a preferred embodiment, Y is ethoxy. In another preferred embodiment, Y is a mixture of ethoxy and propoxy having a molar ratio of ethoxy : propoxy of > 1:1, > 1.1:1, > 1.2:1, > 1.5:1, >2:1, >3:1, or > 5:1.

In a preferred embodiment, m, on a molar average, is from 3 to 100, preferably from 3 to 40, more preferably from 3 to 20, in particular from 3 to 10. In a preferred embodiment, m, on a molar average, is 3, 4, 5, 6, 7, 8, 9 or 10.

In a preferred embodiment, the calcium catalyst comprises or consist of at least one calcium salt which comprises calcium cations and one or more types of anions selected from the group consisting of branched or linear C₈-C₁₂-alkanoate (preferably branched C₈-C₁₂-alkanoate, in particular branched nonanoate), and hydrogensulfate, sulfate.

As used herein, a branched or linear C₈-C₁₂-alkanoate may be any branched or linear alkanoate that has 8, 9, 10, 11 or 12 carbon atoms. An alkanoate is, in the broadest sense, the anion of a carboxylic acid. It, thus, typically has the structure

R^{b}-COO⁻ ,

wherein R^{b} is a branched or linear C₇-C₁₁-alkyl group. In a preferred embodiment, the branched or linear C₈-C₁₂-alkanoate is a branched or linear octanoate, a branched or linear nonanoate, a branched or linear decanoate, a branched or linear undecanoate, or a branched or linear dodecanoate.

In a preferred embodiment, the calcium catalyst comprises or consist of at least one calcium salt of a stoichiometry of formula (II)

(Ca²⁺)ₓ(R1^{p-})_{y}(R2^{q-})_{z} (II),

wherein
x is 1 or 2;
y is 1 or 2;
z is 1 or 2;
p and q are the charges of ions R1 and R2 which are preferably each independently from one another an integer of 0, 1 or 2, in particular 1 or 2, and (yp) + (zq) = (2x); and
R1 and R2 are each independently from one another an anion (anionic ion) as defined herein.

In a preferred embodiment, the calcium catalyst comprises or consists of at least one calcium salt of formula (IIa)

Ca²⁺R1⁻R2⁻ (IIa),

wherein
R1- and R2- are each independently from one another an anion (anionic ion) as defined for R1 and R2 herein and R1- and R2- may optionally also be together a sulfate.

The calcium salt may comprise branched or linear C₈-C₁₂-alkanoate, hydrogensulfate and sulfate in any molar ratio. In a preferred embodiment, the at least one calcium salt comprises branched or linear C₈-C₁₂-alkanoate and the molar ratio branched or linear C₈-C₁₂-alkanoate : calcium is at least 1 : 1. In a preferred embodiment, the at least one calcium salt comprises branched or linear C₈-C₁₂-alkanoate and the molar ratio branched or linear C₈-C₁₂-alkanoate : calcium is at least 1.1 : 1, or is at least 1.5 : 1, or is (approximately) 2 : 1.

In a preferred embodiment, the calcium catalyst comprises or consists of at least one calcium salt which comprises calcium cations and one or more types of anions selected from the group consisting of branched or linear C₈-C₁₂-alkanoate, hydrogensulfate, and sulfate.
In other words, preferably, the at least one calcium salt comprises at least one anion selected from the group consisting of branched or linear C₈-C₁₂-alkanoate, hydrogensulfate and sulfate.
In a preferred embodiment, the at least one calcium salt comprises branched or linear C₈-C₁₂-alkanoate and optionally hydrogensulfate and/or sulfate, wherein, on a molar average, the molar ratio branched or linear C₈-C₁₂-alkanoate : calcium is at least 1 : 1. In a preferred embodiment, the at least one calcium salt comprises branched or linear C₈-C₁₂-alkanoate and optionally hydrogensulfate and/or sulfate, wherein, on a molar average, the molar ratio branched or linear C₈-C₁₂-alkanoate : calcium is at least 1.1 : 1, or is at least 1.5 : 1, or is (approximately) 2 : 1.

In a preferred embodiment, the calcium salt is of formula (II) characterized in that R1 and R2 are each independently an anionic ion selected from the group consisting of branched or linear C₈-C₁₂-alkanoate and sulfate, wherein, on a molar average, at least one of R1 and R2 is branched or linear C₈-C₁₂-alkanoate. In a preferred embodiment, the calcium salt is of formula (IIa)characterized in that R1⁻ and R2⁻ are each independently an anionic ion selected from the group consisting of branched or linear C₈-C₁₂-alkanoate and sulfate, wherein, on a molar average, at least one of R1⁻ and R2⁻ is branched or linear C₈-C₁₂-alkanoate.

The calcium salt may comprise branched or linear C₈-C₁₂-alkanoate (preferably branched C₈-C₁₂-alkanoate, in particular branched nonanoate), hydrogensulfate and sulfate in any molar ratio. The calcium salt may comprise branched or linear C₈-C₁₂-alkanoate (preferably branched C₈-C₁₂-alkanoate, in particular branched nonanoate), hydrogensulfate and sulfate in any molar ratio. In a preferred embodiment, the at least one calcium salt comprises branched or linear C₈-C₁₂-alkanoate (preferably branched C₈-C₁₂-alkanoate, in particular branched nonanoate) and the molar ratio branched or linear C₈-C₁₂-alkanoate (preferably branched C₈-C₁₂-alkanoate, in particular branched nonanoate) : calcium is at least 1 : 1. In a preferred embodiment, the at least one calcium salt comprises branched or linear C₈-C₁₂-alkanoate (preferably branched C₈-C₁₂-alkanoate, in particular branched nonanoate) and the molar ratio branched or linear C₈-C₁₂-alkanoate (preferably branched C₈-C₁₂-alkanoate, in particular branched nonanoate) : calcium is at least 1.1 : 1, or is at least 1.5 : 1, or is (approximately) 2 : 1.

In a preferred embodiment, the calcium catalyst comprises or consists of at least one calcium salt which comprises calcium cations and one or more types of anions selected from the group consisting of branched or linear C₈-C₁₂-alkanoate (preferably branched C₈-C₁₂-alkanoate, in particular branched nonanoate), hydrogensulfate, and sulfate.
In other words, preferably, the at least one calcium salt comprises at least one anion selected from the group consisting of branched or linear C₈-C₁₂-alkanoate (preferably branched C₈-C₁₂-alkanoate, in particular branched nonanoate), hydrogensulfate and sulfate.

In a preferred embodiment, the at least one calcium salt comprises branched or linear C₈-C₁₂-alkanoate (preferably branched C₈-C₁₂-alkanoate, in particular branched nonanoate) and optionally hydrogensulfate and/or sulfate, wherein, on a molar average, the molar ratio branched or linear C₈-C₁₂-alkanoate (preferably branched C₈-C₁₂-alkanoate, in particular branched nonanoate) : calcium is at least 1 : 1. In a preferred embodiment, the at least one calcium salt comprises branched or linear C₈-C₁₂-alkanoate (preferably branched C₈-C₁₂-alkanoate, in particular branched nonanoate) and optionally hydrogensulfate and/or sulfate, wherein, on a molar average, the molar ratio branched or linear C₈-C₁₂-alkanoate (preferably branched C₈-C₁₂-alkanoate, in particular branched nonanoate) : calcium is at least 1.1 : 1, or is at least 1.5 : 1, or is (approximately) 2 : 1.

In a preferred embodiment, the calcium salt is of formula (II) characterized in that R1 and R2 are each independently an anionic ion selected from the group consisting of branched or linear C₈-C₁₂-alkanoate (preferably branched C₈-C₁₂-alkanoate, in particular branched nonanoate) and sulfate, wherein, on a molar average, at least one of R1 and R2 is branched or linear C₈-C₁₂-alkanoate (preferably branched C₈-C₁₂-alkanoate, in particular branched nonanoate). In a preferred embodiment, the calcium salt is of formula (IIa)characterized in that R1⁻ and R2⁻ are each independently an anionic ion selected from the group consisting of branched or linear C₈-C₁₂-alkanoate (preferably branched C₈-C₁₂-alkanoate, in particular branched nonanoate) and sulfate, wherein, on a molar average, at least one of R1⁻ and R2⁻ is branched or linear C₈-C₁₂-alkanoate (preferably C₈-C₁₂-alkanoate, in particular branched nonanoate).

As noted above, nonanoate may be branched or linear. In a preferred embodiment, nonanoate is branched. In a preferred embodiment, nonanoate is branched nonanoate. In a preferred embodiment, the at least one calcium salt comprises anions comprising or consisting of branched nonanoate of formula (V):

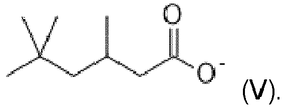

In a preferred embodiment, the calcium salt is of formula (II), characterized in that in the at least one calcium salt of formula (II) R1 and/or R2 is a branched nonanoate residue have the formula (V). In a preferred embodiment, the calcium salt is of formula (II), characterized in that in the at least one calcium salt of formula (IIa), the one or two branched nonanoate residues have the formula (V).

The at least one calcium salt may be obtained by any means. In a preferred embodiment, the at least one calcium salt is obtainable (or obtained) by reacting calcium hydroxide with branched or linear C₈-C₁₂-alkanoic acid which is neutralized with sulfuric acid. In a preferred embodiment, the at least one calcium salt is obtainable (or obtained) by reacting calcium hydroxide with branched nonanoic acid which is neutralized with sulfuric acid.

In a preferred embodiment, the at least one calcium salt is obtainable (or obtained) by reacting calcium hydroxide with branched nonanoic acid of formula (V) which is neutralized with sulfuric acid.

A calcium catalyst may be obtainable (or obtained) from any means in the art. In a preferred embodiment, the calcium catalyst may be obtainable (or obtained) from reacting one or more types of branched or linear C₈-C₁₂-alkanoic acid or a branched or linear C₈-C₁₂-alkanoic salt with a basic calcium salt (e.g., calcium hydroxide, Ca(OH)₂) and subsequently addition of sulfuric acid.

In a preferred embodiment, the calcium catalyst may be obtainable (or obtained) from a method comprising the following steps:
(i) dissolving one or more types of branched or linear C₈-C₁₂-alkanoic acid or a branched or linear C₈-C₁₂-alkanoic salt in a solvent (e.g., water), an alcohol (e.g., propanol) or a mixture thereof;
(ii) adding a basic calcium salt (e.g., calcium hydroxide, Ca(OH)₂) to the mixture of step (i) and, optionally, mixing (e.g., by stirring or shaking);
(iii) adding sulfuric acid to the product of step (ii) and optionally, mixing (e.g., by stirring or shaking).

In a preferred embodiment, the calcium catalyst may be obtainable (or obtained) from a method comprising the following steps:
(i) dissolving one or more types of branched or linear C₈-C₁₂-alkanoate (preferably branched C₈-C₁₂-alkanoate, in particular branched nonanoate) in a solvent (e.g., water, an alcohol (e.g., propanol), or a mixture thereof;
(ii) adding calcium hydroxide to the mixture of step (i) and, optionally, mixing;
(iii) adding sulfuric acid to the product of step (ii).

### (C) Fatty alcohol alkoxylate surfactant

As laid out above, the present invention relates to a fatty alcohol alkoxylate surfactant as defined herein. Therefore, a first aspect relates to a fatty alcohol alkoxylate surfactant, obtainable (or obtained) by reacting at least one fatty alcohol with ethylene oxide or a mixture of ethylene oxide and propylene oxide, wherein the reaction is conducted in the presence of a calcium catalyst. Preferred embodiments and definitions are laid out above

It was surprisingly found that fatty alcohol alkoxylate surfactants that comprise at least one fatty alcohol alkoxylate and at least one calcium catalyst exhibit unexpectedly beneficial technical properties independent on the route of preparation.

Accordingly, a further aspect of the present invention refers to a fatty alcohol alkoxylate surfactant comprising:
(A) at least one fatty alcohol alkoxylate as component A; and
(B) at least one calcium catalyst, preferably a calcium salt as defined herein as component B,
preferably wherein the fatty alcohol alkoxylate surfactant is defined as herein, and preferably wherein the weight ratio A : B is from 98.2 : 1.8 to 99.99 : 0.01, more preferably from 98.5 : 1.5 to 99.9 :0.1 and in particular from 99.0 : 1.0 to 99.8 : 0.2.

It will be understood that all embodiments and definitions as laid out above, also *mutatis mutandis* apply to this aspect.

In a preferred embodiment, the at least one fatty alcohol alkoxylate is defined as above. In a preferred embodiment, the at least one calcium catalyst is defined as above. In a preferred embodiment, the at least one fatty alcohol alkoxylate and the at least one calcium catalyst are both defined as above.

A fatty alcohol alkoxylate surfactant of the present invention is exemplified in the example section below. A fatty alcohol alkoxylate surfactant of the present invention may be stored at various conditions. It may be stored at ambient temperature (typically ranging from 15 to 35 °C, preferably from 18 to 22 °C, in particular it is approximately 20 °C). Alternatively, it may also be stored under cooled condition, e.g., at temperatures ranging below -80 °C, from -80 °C to -20 °C, from -25 °C to 0 °C, from 0 °C to 4 °C, or from 4°C to 15 °C. A fatty alcohol alkoxylate surfactant of the present invention may be stored under conditions allowing it to be in contact with light (e.g., sunlight and/or artificial light) or may be stored in the dark. A fatty alcohol alkoxylate surfactant of the present invention may be stored in large containers having volume of 30 liters or more, above 50 liters, above 100 liters, or above 1000 liters (e.g., in a metal tank or barrel, in a plastic tank or barrel, etc.), or in small containers having volume of less than 30 liters, less than 2 liters, or less than 1 liter (e.g., in gas or plastic bottles).

A fatty alcohol alkoxylate surfactant of the present invention may be used for any purpose. For instance, it may be used a detergent, wetting agent, emulsifier, foaming agent, and/or dispersant. It may be used in a consumer goods product such as, e.g., shampoo, shower gel, toothpaste, laundry formulation, and/or for industrial use such as for production processes including emulsification of immiscible phases.

### (D) Means of preparing a fatty alcohol alkoxylate surfactant

The fatty alcohol alkoxylate surfactant of the present invention may be prepared by any means. Preferably, it comprises reacting the at least one fatty alcohol with ethylene oxide or a mixture of ethylene oxide and propylene oxide, wherein the reaction is catalyzed by a calcium catalyst. It was surprisingly found that such method bears unexpectedly beneficial technical benefits.

Accordingly, a further aspect of the present invention refers to a method for preparing a fatty alcohol alkoxylate surfactant, comprising the steps of:
(i) providing:
   (a) at least one fatty alcohol,
   (b) ethylene oxide or a mixture of ethylene oxide and propylene oxide, and
   (c) at least one calcium catalyst, preferably comprising or consisting of at least one calcium salt as defined herein; and
(ii) reacting the at least one fatty alcohol with the ethylene oxide or a mixture of ethylene oxide and propylene oxide in the presence of the at least one calcium catalyst,
preferably wherein the fatty alcohol alkoxylate surfactant is defined as above.

It will be understood that all embodiments and definitions as laid out above in the context of the fatty alcohol alkoxylate surfactant of the present invention also *mutatis mutandis* apply to the method for preparing such.

As indicated above, the provision of at least one calcium catalyst of step (i) may optionally include the procedural step of reacting one or more types of branched or linear C₈-C₁₂-alkanoic acid or a branched or linear C₈-C₁₂-alkanoic salt with a basic calcium salt (e.g., calcium hydroxide, Ca(OH)₂) and subsequent addition of sulfuric acid. Preferred embodiments in this context are provided above.

Step (ii) may be performed by any means. In a preferred embodiment, step (ii) is conducted by means of procedural steps comprising the following steps:
(ii-a) mixing the at least one fatty alcohol and the at least one calcium catalyst may be mixed (e.g., in an autoclave reactor);
(ii-b) heating the mixture of step (ii-a) to a suitable reaction temperature; and
(ii-c) adding ethylene oxide or a mixture of ethylene oxide and propylene oxide to the heated mixture of step (ii-b).

This allows the fatty alcohol alkoxylate surfactant to be obtained as a liquid under the reaction conditions. Optionally, the obtained fatty alcohol alkoxylate surfactant may be further purified.

The at least one fatty alcohol and the at least one calcium catalyst may be used at any content range. In a preferred embodiment, the weight ratio of (at least one fatty alcohol) : (at least one calcium catalyst) is from 90:10 to 99.999 : 0.001, from 95:5 to 99.995 : 0.005, from 98:2 to 99.99 : 0.01, from 98.2 : 1.8 to 99.99 : 0.01, more preferably from 98.5 : 1.5 to 99.9 :0.1 and in particular from 99.0 : 1.0 to 99.8 : 0.2.

Preferably, step (ii-c) of adding ethylene oxide or a mixture of ethylene oxide and propylene oxide is dosing the ethylene oxide or a mixture of ethylene oxide and propylene oxide slowly and/or stepwise to the heated mixture of step (ii-b).

Step (ii) may be conducted at any temperature. In a preferred embodiment, step (ii) is conducted at a temperature of from 100 to 200 °C, preferably of from 150 to 200 °C.

A method for preparing fatty alcohol alkoxylate surfactant is exemplified in the example section below.

As indicated above, the method of the present invention allows a fatty alcohol alkoxylate surfactant to be obtained that has a narrow distribution of the number n of ethoxy, and optionally propoxy, on the molar average, in the obtained fatty alcohol alkoxylate, in particular wherein in more than 70 mol% of the obtained fatty alcohol alkoxylates, n is in the range from 0.5·n to 1.5·n.

Accordingly, the present invention also refers to a method for preparing a fatty alcohol alkoxylate surfactant having a narrow distribution of the number n of ethoxy, and optionally propoxy, on the molar average, in the obtained fatty alcohol alkoxylate, in particular wherein in more than 70 mol% of the obtained fatty alcohol alkoxylates, n is in the range from 0.5·n to 1.5·n, wherein said method comprises the step s(i) and (ii) as laid out herein.

As indicated above, use of the calcium catalyst is has been surprisingly found to be particularly beneficial for preparation of fatty alcohol alkoxylate surfactants.

Therefore, a further aspect of the present invention refers to the use of a calcium catalyst, preferably a calcium salt as defined herein, for catalyzing the reaction of at least one fatty alcohol with ethylene oxide or a mixture of ethylene oxide and propylene oxide to obtain a fatty alcohol alkoxylate, preferably wherein the fatty alcohol alkoxylate surfactant is defined as above.

It will be understood that all embodiments and definitions as laid out above in the context of the fatty alcohol alkoxylate surfactant and the method of the present invention also *mutatis mutandis* apply to the use of a calcium catalyst for catalyzing the reaction of at least one fatty alcohol with ethylene oxide or a mixture of ethylene oxide and propylene oxide to obtain a fatty alcohol alkoxylate.

In particular, the use may also comprise procedural steps as laid out in the context of the method of the present invention.

The obtainable (or obtained) fatty alcohol alkoxylate surfactant may have any properties as laid out in the context of the present invention.

In a preferred embodiment, the calcium catalyst is used for obtaining a narrow distribution of the number n of ethoxy, and optionally propoxy, on the molar average, in the obtained fatty alcohol alkoxylate, in particular wherein in more than 70 mol% of the obtained fatty alcohol alkoxylates, n is in the range from 0.5·n to 1.5·n.

Thus, it was surprisingly found that the calcium catalyst can be used to prepare a fatty alcohol alkoxylate surfactant having a narrow distribution of the number n of ethoxy, and optionally propoxy, on the molar average, in the obtained fatty alcohol alkoxylate. The present invention also relates to the use of a calcium catalyst for preparing a fatty alcohol alkoxylate surfactant having a narrow distribution of the number n of ethoxy, and optionally propoxy, on the molar average, in the obtained fatty alcohol alkoxylate, in particular wherein in more than 70 mol% of the obtained fatty alcohol alkoxylates, n is in the range from 0.5·n to 1.5·n.

The following examples illustrate the invention and embodiments thereof further. The Examples are not intended to limit the scope of the invention.

### Examples

### Example 1

### Preparation of calcium branched nonanoate catalyst

Branched nonanoic acid (141 g) was dissolved in water (33 g) and isopropanol (430 g). Calcium hydroxide (74 g) was then added into the mixture and homogenized for 5 min. Sulfuric acid (13 g) was added slowly into the mixture. The mixture was then homogenised again for 5 min.

### Example 2

### Synthesis of narrow range oleyl alcohol ethoxylate comprising six ethoxylate residues (EO) on molar average (6EO)

Oleyl alcohol (C_{16/18}-fatty alcohol containing unsaturated C₁₈-fatty alcohol, idonine value 70/75; 1700 g) and calcium branched nonanoate catalyst (as obtained from Example 1; 14 g) were added together into an autoclave reactor. The mixture was heated to 160 °C and then ethylene oxide (1900 g) was dosed slowly into the mixture. The narrow range oleyl alcohol ethoxylate comprising six ethoxylate residues (EO) on molar average (6EO) was obtained as a liquid.

It was surprisingly found that using a calcium catalyst, exemplified as calcium branched nonanoate catalyst, leads to high yields of alkoxylate surfactant.

### Example 3 (Comparative Example)

### Synthesis of broad range oleyl alcohol ethoxylate comprising six ethoxylate residues (EO) on molar average (6EO)

Oleyl alcohol (C_{16/18}-unsaturated C₁₈-fatty alcohol; 1700 g) and sodium hydroxide catalyst (7.7 g) were added together into an autoclave reactor. The mixture was heated to 160 °C and then ethylene oxide (1900 g) was dosed slowly into the mixture. The broad range oleyl alcohol ethoxylate comprising six ethoxylate residues (EO) on molar average (6EO) was obtained as a liquid.

### Example 4

### Characterization of the homologue distribution of fatty alcohol ethoxylates

The homologue distribution of ethoxylate residues (EO) was determined by means of mass spectrometry (MS). A full integration of the MS spectrum was performed and considered as 1. Then, the individual compound peaks were integrated separately. In the present case, the MS spectrum included less than 1% by weight of impurities. The percentage molar content of ethoxylate residues (EO) was determined.

**Table 1. Determined homologue distribution of alcohol ethoxylates in narrow range oleyl alcohol ethoxylate and broad range oleyl alcohol ethoxylate (comparative), wherein both are 6 EO in averaoe**

| Number of EO per oleyl alcohol | Narrow range according to Example 2 (inventive) (in mol%) | Broad range according to Example 3 (comparison) (in mol%) |
|---|---|---|
| 0 (only fatty alcohol) | 3.3 | 9.9 |
| 1 | 1.9 | 7.2 |
| 2 | 3.0 | 8 |
| 3 | 6.1 | 9.1 |
| 4 | 11.5 | 9.6 |
| 5 | 17.9 | 9.1 |
| 6 | 20.7 | 9.1 |
| 7 | 17.4 | 8.5 |
| 8 | 10.6 | 7.7 |
| 9 | 4.7 | 7.6 |
| 10 | 1.6 | 4.1 |
| 11 | 0.4 | 3.8 |
| 12 | 0.1 | 2.6 |
| 13 | 0 | 1.6 |
| 14 | 0 | 0.7 |
| 15 | 0 | 0.3 |
| 16 | 0 | 0.1 |

It was surprisingly found that using a calcium catalyst, exemplified as calcium branched nonanoate catalyst, leads to unexpectedly beneficial technical results with respect to narrow distribution of number of the number of alkoxylate residues. Thus, a calcium catalyst leads to a higher homogeneity of the obtained fatty acid alkoxylate surfactant.

### Example 5

### Liquid detergent formulation

A liquid detergent formulation, comprising:
(A) 88 % by weight, referred to as the liquid detergent formulation, of water; and
(B) 12 % by weight, referred to as the liquid detergent formulation, of a surfactant mixture containing oleyl alcohol ethoxylate (with 6EO as obtained from Example 2 or Example 3, respectively) and oleyl ether sulfate (made by sulfation of the oleyl alcohol ethoxylate of example 2 or example 3 respectively, the sulfation was carried out by reacting the oleyl alcohol ethoxylate with sulfamic acid in molar ratio 1:1),
was prepared. The surfactant mixture was thoroughly mixed with the water. The pH value was adjusted to pH 7. Then, the liquid was allowed to equilibrate for 24 hours at room temperature of approximately 20 °C. The obtained liquid detergent formulation is considered as a potential basis for a liquid laundry detergent formulation.

Subsequently, the phase behaviour was observed. The results are summarized in Table 2 below.

**Table 2. Comparison of liquid detergent formulation. The weight percentages (% by weight) refer to the surfactant mixture containing oleyl alcohol ethoxylate and oleyl ether sulfate**

| Formulation No. | Ether sulfate (% by weight) | Alcohol ethoxylate (% by weight) | Type of alcohol ethoxylate and ether sulfate | Narrow range according to Example 2 (inventive) (in mol%) |
|---|---|---|---|---|
| F1 | 80 | 20 | Narrow range according to Example 2 (inventive) | Liquid |
| F2 | 60 | 40 | Narrow range according to Example 2 (inventive) | Liquid |
| F3 | 50 | 20 | Narrow range according to Example 2 (inventive) | Liquid |
| F4 | 40 | 60 | Narrow range according to Example 2 (inventive) | Liquid |
| C1 | 80 | 20 | Broad range according to Example 3 (comparative) | Liquid |
| C2 | 60 | 40 | Broad range according to Example 3 (comparative) | Two phase liquid |
| C3 | 50 | 20 | Broad range according to Example 3 (comparative) | Two phase liquid |
| C4 | 40 | 60 | Broad range according to Example 3 (comparative) | Two phase liquid |

It was surprisingly found that all liquid detergent formulations F1 to F4 containing a narrow range alcohol ethoxylate and ether sulfate according to Example 2 of the present invention were liquid and exhibit desirable single phase behaviour. In contrast, higher concentrations of broad range alcohol ethoxylate according to Example 3 (comparative), as found for comparative formulations C2 to C4, showed undesirable phase separation into two distinct phases.

Thus, the fatty alcohol alkoxylate surfactant of the present invention is surprisingly beneficial for preparing detergent formulations.

## Claims

1. A fatty alcohol alkoxylate surfactant, obtainable by reacting at least one fatty alcohol with ethylene oxide or a mixture of ethylene oxide and propylene oxide, wherein the reaction is conducted in the presence of a calcium catalyst.

2. The fatty alcohol alkoxylate surfactant according to claim 1, **characterized in that** the calcium catalyst comprises or consists of at least one calcium salt which comprises calcium cations and one or more types of anions selected from the group consisting of branched or linear C₈-C₁₂-alkanoate, preferably branched C₈-C₁₂-alkanoate, in particular branched nonanoate, hydrogensulfate, sulfate, an anionic ion of formula (III), and an anionic ion of formula (IV) 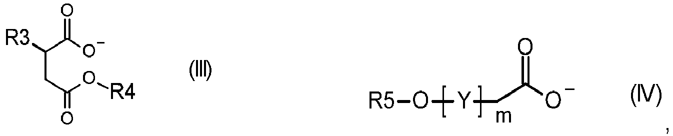 wherein
R3 is a linear or branched alkyl group having 1 to 20 carbon atoms or a linear or branched alkenyl group having 2 to 20 carbon atoms, preferably a linear or branched alkyl or alkenyl group having 6 to 18 carbon atoms, in particular a linear or branched alkyl or alkenyl group having 8 to 16 carbon atoms;
R4 is a linear or branched alkyl group having 1 to 20 carbon atoms or a linear or branched alkenyl group having 2 to 20 carbon atoms, preferably a linear or branched alkyl group having 1 to 16 carbon atoms or a linear or branched alkenyl group having 2 to 16 carbon atoms, in particular a linear or branched alkyl group having 1 to 12 carbon atoms or a linear or branched alkenyl group having 2 to 12 carbon atoms;
R5 is a linear or branched alkyl group having 1 to 20 carbon atoms or a linear or branched alkenyl group having 2 to 20 carbon atoms, preferably a linear or branched alkyl or alkenyl group having 6 to 20 carbon atoms, in particular a linear or branched alkyl or alkenyl group having 10 to 20 carbon atoms;
Y is selected from ethoxy and mixtures of ethoxy and propoxy, wherein a mixture of ethoxy and propoxy has molar ratio of ethoxy : propoxy of > 1:1, in particular is ethoxy; and
m on a molar average, is from 3 to 100, preferably from 3 to 40, more preferably from 3 to 20, in particular from 3 to 10.

3. The fatty alcohol alkoxylate surfactant according to one or more of claims 1 or 2, **characterized in that** it comprises at least one fatty alcohol alkoxylate described in the following formula (I) 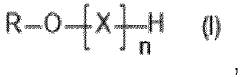 wherein:
R is a linear or branched alkyl or alkenyl group having 6 to 20 carbon atoms, preferably a linear or branched alkyl or alkenyl group having 8 to 20 carbon atoms, more preferably a linear or branched alkyl or alkenyl group having 12 to 20 carbon atoms, even more preferably a linear or branched alkyl or alkenyl group having 16 to 18 carbon atoms, in particular a linear alkyl or alkenyl group having 16 to 18 carbon atoms;
X is selected from ethoxy and mixtures of ethoxy and propoxy, wherein a mixture of ethoxy and propoxy has molar ratio of ethoxy : propoxy of > 1:1, in particular is ethoxy;
n on a molar average, is from 3 to 100, preferably from 3 to 60, more preferably from 3 to 40, even more preferably from 4 to 20, in particular from 4 to 15.

4. The fatty alcohol alkoxylate surfactant according to claim 3, **characterized in that** in the at least one fatty alcohol alkoxylate of formula (I):
R is a linear alkyl or alkenyl group having 16 to 18 carbon atoms;
X is ethoxy; and
n on a molar average, is from 4 to 20, in particular from 4 to 15,
in particular wherein in more than 70 mol% of the fatty alcohol alkoxylates, n is in the range from 0.5·n to 1.5·n.

5. The fatty alcohol alkoxylate surfactant according to one or more of claims 1 to 4, **characterized in that** it comprises a fatty alcohol alkoxylate, preferably the one or more fatty alcohol alkoxylate of formula (I), in an amount from 0.1 to 99.99% by weight, preferably in an amount from 1 to 99.9% by weight, more preferably in an amount from 10 to 99.9% by weight and even more preferably in an amount from 50 to 99.9%, in each case based on the total weight of the fatty alcohol alkoxylate surfactant.

6. The fatty alcohol alkoxylate surfactant according to one or more of claims 2 to 5, **characterized in that** the at least one calcium salt comprises branched nonanoate and optionally hydrogensulfate and/or sulfate, wherein, on a molar average, the molar ratio branched nonanoate : calcium is at least 1 : 1.

7. The fatty alcohol alkoxylate surfactant according to one or more of claims 2 to 6, **characterized in that** the at least one calcium salt comprises anions comprising or consisting of branched nonanoate of formula (V):

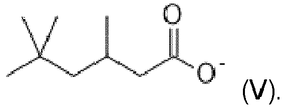

8. The fatty alcohol alkoxylate surfactant according to one or more of claims 2 to 7, **characterized in that** the at least one calcium salt is obtainable by reacting calcium hydroxide with branched or linear C₈-C₁₂-alkanoic acid which is neutralized with sulfuric acid.

9. The fatty alcohol alkoxylate surfactant according to one or more of claims 1 to 8, **characterized in that** it comprises the one or more calcium catalysts in an amount from 0.01 to 10% by weight, preferably in an amount from 0.01 to 5% by weight, more preferably in an amount from 0.05 to 3% by weight and even more preferably in an amount from 0.1 to 2%, in each case based on the total weight of the fatty alcohol alkoxylate surfactant.

10. The fatty alcohol alkoxylate surfactant according to one or more of claims 1 to 9, wherein the mixture of ethylene oxide and propylene oxide has molar ratio of (ethylene oxide) : (propylene oxide) of > 1:1.

11. A fatty alcohol alkoxylate surfactant comprising:
(A) at least one fatty alcohol alkoxylate as component A; and
(B) at least one calcium catalyst, preferably a calcium salt as defined in one or more claims 2, 6, 7 or 8 as component B,
preferably wherein the fatty alcohol alkoxylate surfactant is defined as in one or more of claims 1 to 10, and
preferably wherein the weight ratio A : B is from 98.2 : 1.8 to 99.99 : 0.01, more preferably from 98.5 : 1.5 to 99.9 :0.1 and in particular from 99.0 : 1.0 to 99.8 : 0.2.

12. A method for preparing a fatty alcohol alkoxylate surfactant, comprising the steps of:
(i) providing:
(a) at least one fatty alcohol,
(b) ethylene oxide or a mixture of ethylene oxide and propylene oxide, and
(c) at least one calcium catalyst, preferably comprising or consisting of at least one calcium salt as defined in one or more claims 2, 6, 7 or 8; and
(ii) reacting the at least one fatty alcohol with the ethylene oxide or a mixture of ethylene oxide and propylene oxide in the presence of the at least one calcium catalyst,
preferably wherein the fatty alcohol alkoxylate surfactant is defined according to one or more of claims 1 to 11.

13. The method according to claim 12, wherein step (ii) is conducted at a temperature of from 100 to 200 °C, preferably of from 150 to 200 °C.

14. The use of a calcium catalyst, preferably a calcium salt as defined in one or more claims 2, 6, 7 or 8, for catalyzing the reaction of at least one fatty alcohol with ethylene oxide or a mixture of ethylene oxide and propylene oxide to obtain a fatty alcohol alkoxylate, preferably wherein the fatty alcohol alkoxylate surfactant is defined according to one or more of claims 1 to 11.

15. The use according to claim 14, **characterized in that** the calcium catalyst is used for obtaining a narrow distribution of the number n of ethoxy, and optionally propoxy, on the molar average, in the obtained fatty alcohol alkoxylate, in particular wherein in more than 70 mol% of the obtained fatty alcohol alkoxylates, n is in the range from 0.5·n to 1.5·n.
